# EUROPÄISCHE PATENTSCHRIFT

(11) **EP 1 575 464 B1**
(45) Veröffentlichungstag und Bekanntmachung des Hinweises auf die Patenterteilung: **14.06.2006**
(21) Anmeldenummer: 03813543.0
(22) Anmeldetag: 13.02.2003
(51) Int. Cl.: A61F 5/01

(54) **VORRICHTUNG ZUM AUFBRINGEN EINER VENTRAL ODER DORSAL GERICHTETEN TRANSLATIONSKRAFT IM KNIEGELENKSBEREICH**
DEVICE FOR APPLYING A VENTRALLY OR DORSALLY DIRECTED TRANSLATORY FORCE IN THE AREA OF A KNEE JOINT
DISPOSITIF DESTINE A APPLIQUER UNE FORCE DE TRANSLATION DIRIGEE DE FACON VENTRALE OU DORSALE DANS LA ZONE DE L'ARTICULATION DU GENOU

(30) Priorität: 19.12.2002 DE 10259751
(43) Veröffentlichungstag der Anmeldung: 21.09.2005
(73) Patentinhaber: Albrecht GmbH, 83115 Neubeuern (DE)
(72) Erfinder: ALBRECHT, Erich, 83115 Neubeuern (DE); OPAHLE, Hans-Georg, 83024 Rosenheim (DE)
(74) Vertreter: Bauer, Friedrich
(86) Internationale Anmeldenummer: PCT/EP2003/001456
(87) Internationale Veröffentlichungsnummer: WO 2004/056293

(56) Entgegenhaltungen:
- EP-A- 1 114 619
- DE-U- 29 705 405

## Beschreibung

Die Erfindung betrifft eine Vorrichtung zum Aufbringen einer ventral oder dorsal gerichteten Translationskraft auf einen Unterschenkel im Bereich eines Kniegelenks zur Behandlung oder Nachbehandlung einer Knieinstabilität, insbesondere Kreuzbandinstabilität, gemäß dem Oberbegriff des Anspruches 1.

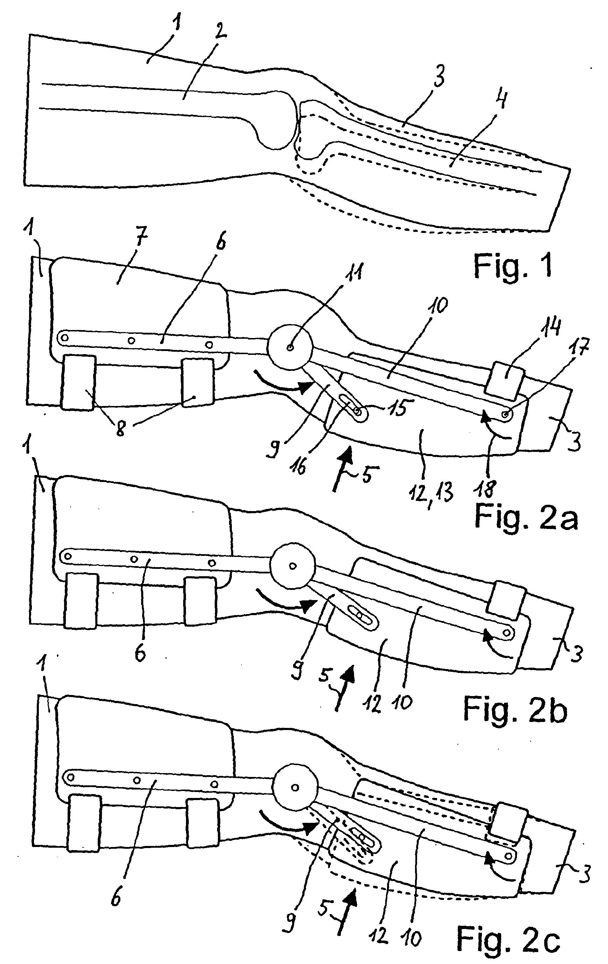

Auf dem Gebiet von Knieinstabilität bereiten insbesondere hintere Kreuzbandläsionen große medizinische Probleme. Obwohl es ausgereifte chirurgische Rekonstruktionsverfahren für hintere Kreuzbandrupturen und andere Läsionen der hinteren Kreuzbänder gibt, wird das operative Ergebnis häufig nach einigen Monaten wieder dadurch verschlechtert oder völlig zunichte gemacht, dass das genähte oder rekonstruierte Band aufgrund der Schwerkraft des Unterschenkels, die bei einem auf dem Rücken liegenden Patienten in dorsaler Richtung wirkt, in unerwünschter Weise wieder gedehnt wird. Hierdurch kommt es zu einer Fehlstellung der Tibia zum Femur, wie in Figur 1 in gestrichelten Linien dargestellt ist. Die durchgezogenen Linien in Figur 1 zeigen dagegen die Tibia und den Unterschenkel im Normalzustand. Die Gefahr einer derartigen Fehlstellung, d.h. des Absenkens der Tibia relativ zum Femur, besteht selbstverständlich auch dann, wenn eine hintere Kreuzbandläsion nicht operativ, sondern konservativ behandelt wird.

Bei einer vorderen Kreuzbandinstabilität kann das Problem auftreten, dass die Tibia relativ zum Femur zu weit ventral zu liegen kommt. Dies ist insbesondere dann der Fall, wenn die Tibiagelenkfläche des Patienten so geneigt ist, dass bei aufrechtem Stand, bei dem das Femur auf die schräge Tibiagelenkfläche drückt, auf die Tibia eine Kraft in ventraler Richtung ausübt. Ist das vordere Kreuzband instabil, wird dadurch die Tibia im Kniegelenkbereich in unerwünschter Weise nach vorn, d.h. in Figur 1 nach oben verschoben.

Aus der Patentschrift EP1114619 ist bereits eine Vorrichtung zum Aufbringen einer ventral oder dorsal gerichteten Translationskraft auf den Unterschenkel im Bereich des Kniegelenks bekannt, die dieser unerwünschten Verlagerung der Tibia entgegenwirken soll. Diese bekannte Vorrichtung weist medial und lateral im Bereich des Oberschenkels angeordnete Oberschenkelschienen auf, die mittels einer Manschette am Oberschenkel befestigt werden können. Am distalen Ende der Oberschenkelschienen, die sich seitlich neben dem Knie befinden, ist ein Schwenkhebel gelenkig angebracht, welcher mittels einer Feder entweder in ventraler oder dorsaler Richtung vorgespannt ist. Dieser Schwenkhebel unter- oder übergreift einen seitlich von einer Unterschenkelmanschette abstehenden Bolzen in einem knienahen Bereich des Unterschenkels. Je nachdem, wie die Vorspannung der Feder gerichtet ist, wird hierdurch das obere Ende der Tibia entweder in ventraler oder dorsaler Richtung gedrängt, um einer unerwünschten Dehnung eines lädierten hinteren bzw. vorderen Kreuzbandes und einer damit verbundenen translatorischen Verschiebung der Tibia in dorsaler bzw. ventraler Richtung entgegenzuwirken. Problematisch ist bei dieser bekannten Vorrichtung jedoch, dass durch die Federkraft auf den Unterschenkel eine permanente Kraft in Extensions- bzw. Flexionsrichtung ausgeübt wird, d.h. ein Drehmoment, welches den Unterschenkel permanent zu strecken bzw. zu beugen versucht. Ein freies, ungehindertes Strecken bzw. Beugen des Knies ist daher mit dieser bekannten Vorrichtung nicht möglich.

Ausgehend hiervon, liegt der Erfindung die Aufgabe zugrunde, eine Vorrichtung der eingangs genannten Art zu schaffen, welche auf besonders wirkungsvolle Weise eine unerwünschte Verschiebung der Tibia in dorsaler oder ventraler Richtung verhindert und eine freie Beweglichkeit des Kniegelenks ermöglicht.

Diese Aufgabe wird erfindungsgemäß durch die Merkmale des Anspruches 1 gelöst. Vorteilhafte Ausführungsformen der Erfindung sind in den weiteren Ansprüchen beschrieben.

Die erfindungsgemäße Vorrichtung weist folgende Merkmale auf:
- die Unterschenkelschieneneinrichtung weist einen kürzeren und einen längeren Schienenarm auf, wobei beide Schienenarme relativ zur Oberschenkelschiene schwenkbar sind,
- die beiden Schienenarme sind relativ zueinander schwenkbar angeordnet,
- der kürzere Schienenarm ist an seinem distalen Ende mit der Fixiereinrichtung in einem knienahen Bereich in Kopplungseingriff, während der längere Schienenarm mit seinem distalen Ende mit der Fixiereinrichtung in einem knieferneren Bereich in Kopplungseingriff ist,
- die Vorspannkraft der Federeinrichtung wirkt zwischen dem kürzeren und längeren Schienenarm derart, dass die Schienenarme dazu gedrängt werden, relativ zueinander eine Schwenkbewegung auszuführen, so dass auf die Fixiereinrichtung im knienahen Bereich eine ventral oder dorsal gerichtete Translationskraft aufgebracht wird.

Für die erfindungemäße Vorrichtung ist somit charakteristisch, dass die Federkraft nicht zwischen der Oberschenkelschiene und der Unterschenkelschieneneinrichtung wirkt, sondern zwischen den beiden Schienenarmen, die sich vom Kniegelenksbereich aus in distaler Richtung neben dem Unterschenkel erstrecken und aufgrund ihrer unterschiedlichen Längen mit ihren distalen Enden an unterschiedlichen Stellen mit der Unterschenkel-Fixiereinrichtung in Kopplungseingriff sind. Hierdurch wird auf die Fixiereinrichtung und damit auf den Unterschenkel ein Drehmoment ausgeübt, das die Tibia im kniegelenksnahen Bereich je nach Richtung der Federkraft und Anordnung der Schienenarme entweder in die ventrale oder dorsale Richtung drängt. Beide Unterschenkel-Schienenarme sind dagegen am distalen. Ende der Oberschenkelschiene frei schwenkbar relativ zu Oberschenkelschiene gelagert, d.h. dass keine Federkraft zwischen der Oberschenkelschiene einerseits und den beiden unteren Schienenarmen andererseits wirkt. Der Patient kann daher sein Knie ohne zusätzlichen Kraftaufwand beugen oder strecken.

Gemäß einer vorteilhaften Ausführungsform weist mindestens einer der Schienenarme an seinem distalen Ende ein Langloch auf, in das ein Bolzen der Fixiereinrichtung hineinragt, um den Schienenarm längsverschiebbar mit der Fixiereinrichtung zu koppeln. Hierdurch wird dem Umstand Rechnung getragen, dass sich die Abstände zwischen der Schwenkachse der Vorrichtung und den beiden Kopplungspunkten, an denen die Unterschenkel-Schienenarme an der Fixiereinrichtung angreifen, in Abhängigkeit der Schwenkposition der Fixiereinrichtung variieren können. Die längsverschiebbare Kopplung des mindestens einen Schienenarmes an der Fixiereinrichtung behindert somit nicht die Schwenkbewegung der Fixiereinrichtung mittels der beiden Schienenarme. Alternativ zu einem Langloch wäre es jedoch auch ohne weiteres möglich, den betreffenden Schienenarm nur von einer Seite her an den betreffenden Bolzen anzulegen, so dass dieser Schienenarm von einer Seite her gegen den Bolzen drückt.

Zweckmäßigerweise besteht die am Unterschenkel befestigbare Fixiereinrichtung aus einer Halbschale, wobei die beiden Schienenarme mit der Halbschale in den beiden gegenüberliegenden Endbereichen der Halbschale gekoppelt sind.

Gemäß einer vorteilhaften Ausführungsform umfasst die Federeinrichtung eine Spiralfeder, die in einem Federgehäuse angeordnet ist, das an einem der Schienenarme befestigt und zusammen mit diesem relativ zur Oberschenkelschiene verschwenkbar ist. Zweckmäßigerweise fällt hierbei die Mittelachse des Federgehäuses mit der Schwenkachse zusammen. Auf diese Weise lässt sich eine besonders einfache und platzsparende Ausführungsform realisieren.

Weiterhin ist es zweckmäßig, wenn zur Einstellung der gewünschten Translationskraft die Vorspannkraft der Federeinrichtung mittels eines Zahnradgetriebes einstellbar ist, das sich im Federgehäuse befindet.

Gemäß einer vorteilhaften Ausführungsform ist die Unterschenkelschieneneinrichtung in einem Langloch der Oberschenkelschiene verschiebbar an der Oberschenkelschiene gelagert. Hierdurch kann die Unterschenkelschieneneinrichtung zusätzlich zur Schwenkbewegung eine translatorische Bewegung relativ zur Oberschenkelschiene ausüben und dadurch dem Bewegungsweg beim Beugen und Strecken des Knies optimal folgen, ohne dass es zu unangenehmen Zug- oder Druckspannungen an der Oberschenkelmanschette längs des Oberschenkels kommt. Zweckmäßigerweise erstreckt sich hierzu das Langloch in Längsrichtung der Oberschenkelschiene.

Die Erfindung wird nachfolgend anhand der Zeichnungen beispielhaft näher erläutert. Es zeigen:
- Figur 1:: eine schematische Darstellung des Beins eines Menschen im Bereich des Kniegelenks mit Femur und Tibia, wobei Unterschenkel und Tibia in der normalen Stellung und einer dorsal verschobenen Stellung dargestellt sind,
- Figuren 2a bis 2c:: Seitenansichten einer am Bein angelegten ersten Ausführungsform der erfindungsgemäßen Vorrichtung in verschiedenen Winkelstellungen der Unterschenkel-Schienenarme zur Verdeutlichung der Funktion der Erfindung,
- Figur 3:: eine Explosionsdarstellung der wichtigsten Einzelteile der Vorrichtung von Figur 2a bis 2c,
- Figur 4:: einen Querschnitt durch die erfindungsgemäße Vorrichtung im Bereich des Federgehäuses,
- Figuren 5a, 5b:: eine Draufsicht bzw. Seitenansicht des Antriebszahnrades zum Spannen der Feder,
- Figuren 6a, 6b:: eine Draufsicht bzw. Seitenansicht des Zahnrades zum Spannen der Feder,
- Figuren 7 bis 9:: eine Draufsicht auf die erfindungsgemäße Vorrichtung von den Ebenen VII, VIII bzw. IX aus, wie in Figur 4 eingezeichnet,
- Figur 10:: eine Seitenansicht einer zweiten Ausführungsform der erfindungsgemäßen Vorrichtung, wobei sich die Vorrichtung weitgehend in einer Extensionsstellung befindet,
- Figur 11:: die Vorrichtung von Figur 10 in Flexionsstellung

- Figur 12:: eine Seitenansicht und einen Längsschnitt durch die bei der zweiten Ausführungsform verwendete Mutter, und
- Figur 13:: eine verkürzte Seitenansicht der bei der zweiten Ausführungsform verwendeten Oberschenkelschiene mit Langloch.

Aus Figur 1 ist ein Oberschenkel 1 mit einem Femur 2 sowie ein Unterschenkel 3 mit einer Tibia 4 ersichtlich. Hierbei ist mit durchgezogenen Linien die Normallage und mit gestrichelten Linien eine dorsal abgesenkte Lage des Unterschenkels 3 und der Tibia 4 dargestellt, die bei Kreuzbandinstabilitäten aufgrund der dorsal wirkenden Schwerkraft bei einem auf dem Rücken liegenden Patienten entstehen kann.

Aus den Figuren 2a bis 2c ist eine Vorrichtung in Form einer Orthese ersichtlich, mit der auf den Unterschenkel 3 in einem knienahen Bereich eine der Tibiaabsenkung entgegenwirkende, ventral gerichtete Translationskraft aufgebracht werden kann, um eine Absenkung der Tibia 4 auch bei geschwächten Kreuzbändern von vornherein nicht entstehen zu lassen oder eine Absenkung rückgängig zu machen. Die Richtung der durch die Orthese aufgebrachten Translationskraft ist hierbei durch den Pfeil 5 gekennzeichnet. Figur 2a zeigt die an einem Bein angelegte Orthese, wobei sich der Unterschenkel 3 in einem abgesenkten Zustand befindet. Aus Figur 2b ist wiederum die normale Unterschenkellage ersichtlich, die sich aufgrund der durch die Orthese aufgebrachten Translationskraft einstellt. Figur 2c zeigt nochmals die in den Figuren 2a und 2b gezeigten Lagen des Unterschenkels 3 und der Orthese mit gestrichelten bzw. durchgezogenen Linien zum direkten Vergleich der beiden Zustände.

In den Figuren 2a bis 2c ist nur die sich auf einer Seite des Beins befindende Schienenanordnung ersichtlich. Die gesamte Orthese besteht jedoch aus zwei derartigen Schienenanordnungen, die lateral und medial neben dem Bein verlaufen und spiegelbildlich zueinander angeordnet sind. Es ist jedoch auch denkbar, dass eine derartige Schienenanordnung nur auf einer Seite des Beins vorgesehen wird.

Aus den Figuren 2a bis 2c ist eine Oberschenkelschiene 6 ersichtlich, die mittels einer Manschette 7 am Oberschenkel 1 befestigbar ist. Zweckmäßigerweise besteht die Manschette 7 aus einer auf die Vorderseite des Oberschenkels aufgelegten Halbschale, die mittels um die Rückseite des Oberschenkels herumgeführter, Klettverschlüsse aufweisender Bänder 8 am Oberschenkel 1 fixierbar ist. Im distalen Endbereich der Oberschenkelschiene 6, der sich seitlich neben dem Kniegelenk befindet, sind ein kürzerer Schienenarm 9 und ein längerer Schienenarm 10 schwenkbar gelagert, die sich in Richtung des Unterschenkels 3 erstrecken. Die Schwenkachse ist mit 11 gekennzeichnet. Beide Schienenarme 9, 10, die zusammen hier als Unterschenkelschieneneinrichtung bezeichnet werden, sind relativ zur Oberschenkelschiene 6 frei schwenkbar, d.h. es wirkt zwischen der Oberschenkelschiene 6 einerseits und der Unterschenkelschieneneinrichtung andererseits keine Federkraft. Dagegen ist zwischen dem kürzeren Schienenarm 9 und dem längeren Schienenarm 10 eine Federkraft wirksam, welche den kürzeren Schienenarm 9 im Gegenuhrzeigersinn und den längeren Schienenarm 10 im Uhrzeigersinn zu drehen versucht.

Die beiden distalen Enden der Schienenarme 9, 10 wirken auf eine Fixiereinrichtung 12 ein, die aus einer dorsal, d.h. im Wadenbereich angeordneten Halbschale 13 besteht, die sich von einem knienahen Bereich längs des Unterschenkels 3 in distaler Richtung erstreckt. Die Halbschale 13 ist mittels eines einen Klettverschluß aufweisenden Haltebands 14 am Unterschenkel 3 befestigt.

Das distale Ende des kürzeren Schienenarms 9 ist, wie aus den Figuren 2a bis 2c ersichtlich, mit einem Bolzen 15 in Kopplungseingriff, der sich im knienahen Endbereich der Fixiereinrichtung 12 von dieser aus seitlich nach außen erstreckt. Hierzu weist der kürzere Schienenarm 9 ein Langloch 16 auf, in das der Bolzen 15 eingreift. Der Schienenarm 9 ist aufgrund dieses Langlochs 16 somit längsverschiebbar am Bolzen 15 geführt.

Der längere Schienenarm 10 ist an seinem distalen Ende an einem Bolzen 17 in einem distalen Endbereich der Fixiereinrichtung 12 schwenkbar angelenkt. Die Halbschale 13 lässt sich somit um den Bolzen 17 schwenken, wie durch den Pfeil 18 angedeutet. Aufgrund des Abstandes der Bolzen 15 und 17 in Längsrichtung des Unterschenkels 3 führt somit die Halbschale 13 eine Schwenk- bzw. Kippbewegung im Uhrzeigersinn aus und übt dadurch die gewünschte Translationskraft in ventraler Richtung auf die Tibia 4 aus.

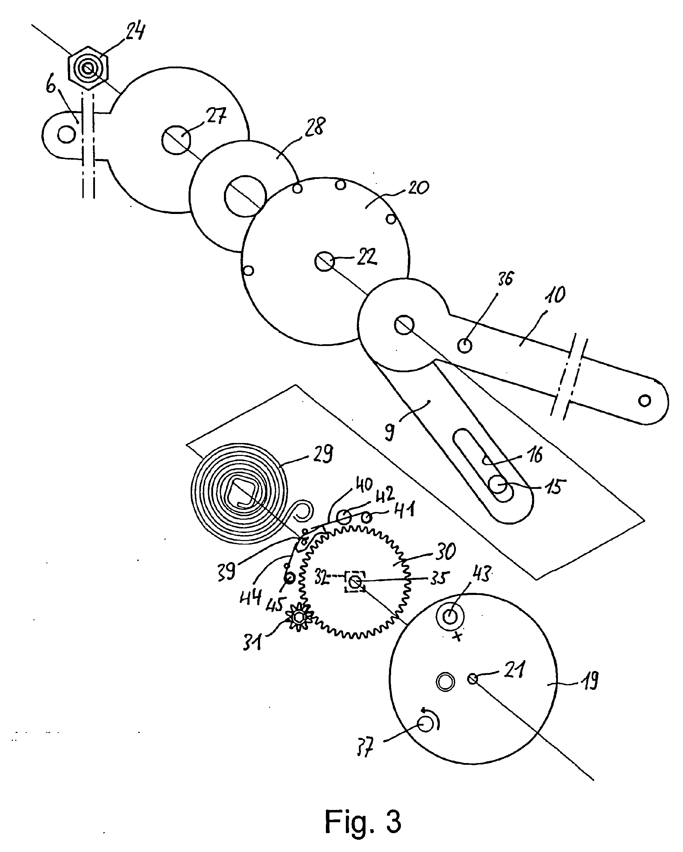

Anhand von Figur 3 bis 9 wird im Folgenden der Aufbau der in den Figuren 2a bis 2c gezeigten Schienenanordnung näher erläutert.

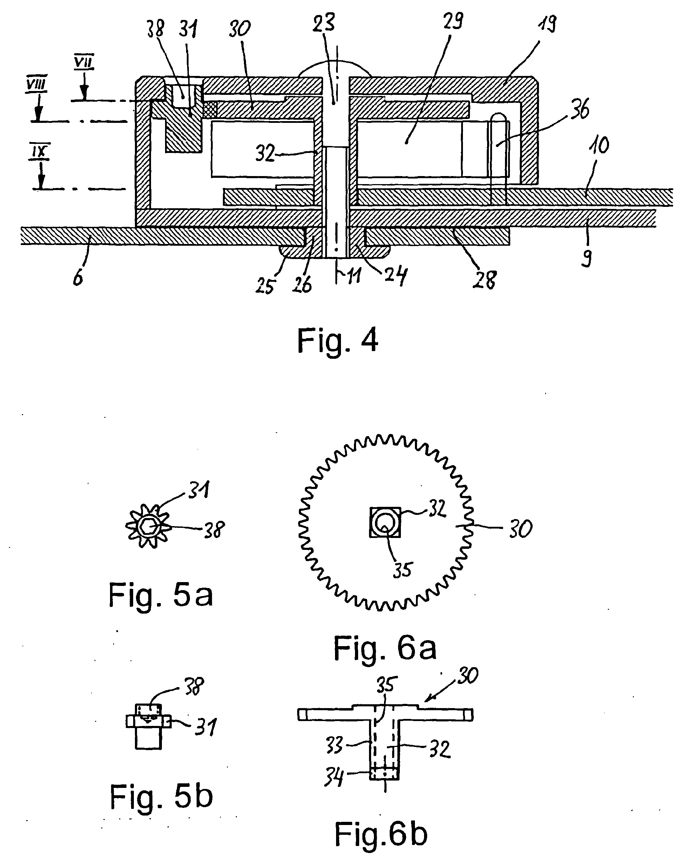

Diese Schienenanordnung weist, wie aus den Figuren 3 und 4 ersichtlich, im Schienengelenksbereich ein napfförmiges Federgehäuse 19 auf, das auf dem proximalen Endbereich des kürzeren Schienenarms 9 aufgeschraubt und daher fest mit diesem verbunden ist.

Der proximale Endbereich des kürzeren Schienenarms 9 ist als Kreisscheibe 20 ausgebildet, deren Außendurchmesser dem Außendurchmesser des Federgehäuses 19 entspricht. Das Federgehäuse 19 und der Schienenarm 9 weisen jeweils ein mittiges Durchgangsloch 21 bzw. 22 auf, durch die eine Schraube 23 hindurchgeführt werden kann. Die Schraube 23 ist mit einem Gewinde versehen, auf das eine Mutter 24 aufgeschraubt werden kann, bis diese am Schienenarm 9 anliegt. Die Schraube 23 und die Mutter 24 sind damit relativ zum Federgehäuse 19 und Schienenarm 9 festgelegt.

Die Mutter 24 weist einen sechseckigen Kopf 25 und einen axial vorstehenden zylinderischen Bund 26 auf, der einen kleineren Außendurchmesser als der Kopf 25 hat. Dieser zylinderische Bund 26 dient als Drehlager für die Oberschenkelschiene 6, die zu diesem Zweck in ihrem distalen Endbereich ein mittiges Durchgangsloch 27 aufweist, dessen Durchmesser auf den Außendurchmesser des zylinderischen Bunds 26 abgestimmt ist. Zwischen der Oberschenkelschiene 6 und dem kürzen Schienenarm 9 ist eine reibungsvermindernde Scheibe 28, insbesondere aus Teflon, angeordnet. Es ist erkennbar, dass aufgrund dieser Anordnung der kürzere Schienenarm 9 zusammen mit dem Federgehäuse 19 frei um die Schwenkachse 11 geschwenkt werden kann.

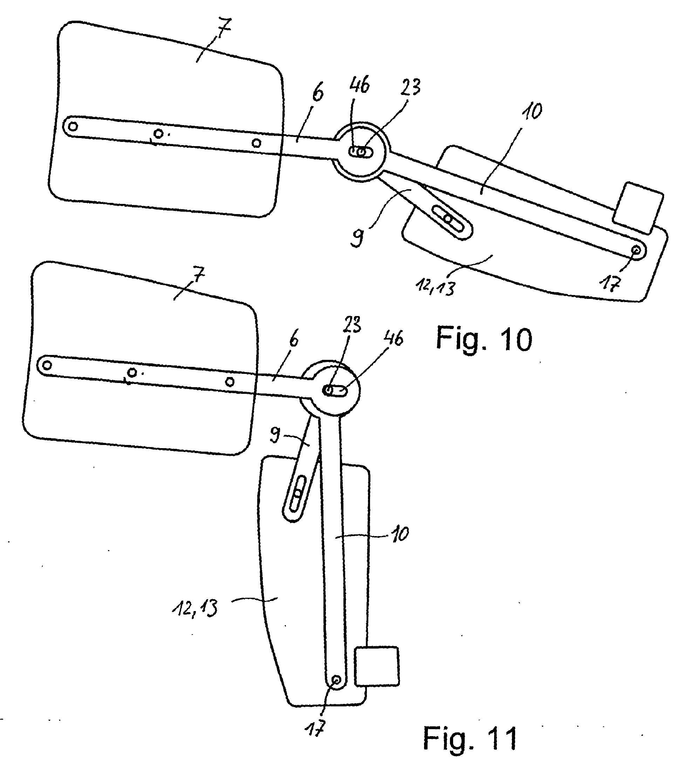

Innerhalb des Federgehäuses 19 ist, wie aus den Figuren 3 und 4 ersichtlich, eine Feder in Form einer Spiralfeder 29 angeordnet, mit der eine zwischen dem kürzeren Schienenarm 9 und dem längeren Schienenarm 10 wirkende Vorspannkraft erzeugt wird. Diese Spiralfeder 29 wirkt, wie nachfolgend noch näher erläutert wird, mit einem ebenfalls innerhalb des Federgehäuses 19 angeordneten Zahnradgetriebe zusammen, das aus einem zentralen Zahnrad 30 und einem mit diesem kämmenden, peripheren Antriebszahnrad 31 kleineren Durchmessers besteht. Das zentrale Zahnrad 30, das in den Figuren 6a, 6b näher dargestellt ist, weist einen mittigen, axialen Fortsatz 32 auf, der über den überwiegenden Teil seiner Länge, d.h. im Bereich 33, außen einen viereckigen Querschnitt hat. Der Endbereich 34 des Fortsatzes 32 ist dagegen außen zylindrisch ausgeführt und dient als Drehlager für den längeren Schienenarm 10 (siehe Figur 10). Der Schienenarm 10, der sich durch eine Aussparung in der Umfangswand des Federgehäuses 19 hindurch erstreckt, ist somit um den Fortsatz 32 herum schwenkbar.

Das zentrale Zahnrad 30 weist weiterhin eine mittige, axiale Durchgangsbohrung 35 auf, durch welche die Schraube 23 hindurchgeführt werden kann. Die Schraube 23 dient damit als Drehlager für das zentrale Zahnrad 30.

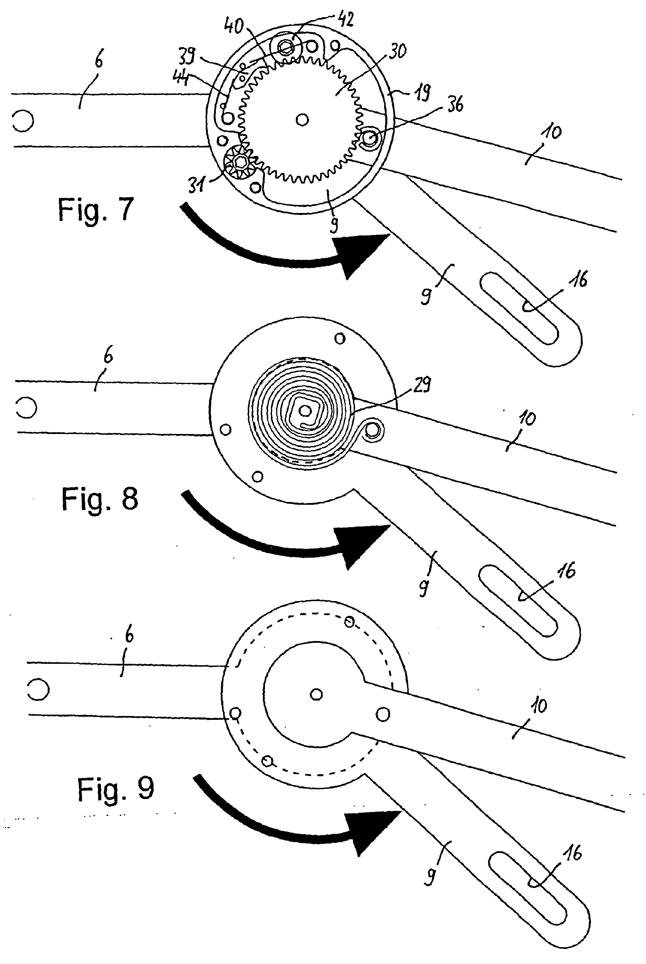

Wie aus den Figuren 3 und 8 ersichtlich, ist die Spiralfeder 29 in ihrem inneren Endbereich derart viereckig gebogen, dass sie mit geringem Spiel auf den viereckigen Fortsatz 32 des zentralen Zahnrads 30 aufgesteckt werden kann. Das innere Ende der Spiralfeder 29 ist damit drehfest am Fortsatz 32 festgelegt. Das äußere Ende der Spiralfeder 29 ist mit einem axial angeordneten Kopplungsstift 36 verbunden, der am längeren Schienenarm 10 befestigt ist.

Aufgrund dieser Anordnung wirkt somit die Vorspannkraft der Feder 29 zwischen dem zentralen Zahnrad 30 und dem längeren Schienenarm 10. Die Vorspannkraft der Feder 29 kann dabei durch ein Verdrehen des Zahnrades 30 verändert werden, was über das Antriebszahnrad 31 erfolgt. Das Verdrehen des Antriebszahnrades 31 erfolgt manuell mittels eines Inbusschlüssels, der durch eine Bohrung 37 im Federgehäuse 19 hindurch eingeführt und mit einer inbusförmigen Aussparung 38 im Antriebszahnrad 31 in Eingriff gebracht werden kann.

Ein unerwünschtes Verdrehen des zentralen Zahnrades 30 relativ zum Federgehäuse 19 wird mittels einer Sperrklinke 39 (Figuren 3 und 7) verhindert, die mit der Verzahnung des Zahnrades 30 in Eingriff ist. Die Sperrklinke 39 ist in nicht näher dargestellter Weise im Federgehäuse 19 schwenkbar gelagert. Eine Federzunge 40, die an einem Haltebolzen 41 des Federgehäuses 19 festgelegt ist, drückt von außen derart auf die Sperrklinke 39, dass deren freies Ende im Normalfall in Eingriff mit der Verzahnung des Zahnrades 30 bleibt und ein Zurückdrehen des Zahnrades 30 aufgrund der Vorspannkraft der Spiralfeder 29 verhindert. Die Federzunge 40 kann jedoch mittels eines Exzenters 42 von der Sperrklinke 39 abgehoben werden, wobei der Exzenter 42 durch eine Öffnung 43 im Federgehäuse 19 hindurchragt und dadurch manuell betätigt werden kann. Wird die Federzunge 40 von der Sperrklinke 39 abgehoben, kann eine weitere Federzunge 44, welche von außen auf den gegenüberliegenden Endbereich der Sperrklinke 39 drückt, die Sperrklinke 39 außer Eingriff mit der Verzahnung bringen,so dass das Zahnrad 30 zurückgedreht und die Vorspannkraft verringert werden kann. Die Federzunge.44 ist ebenfalls mittels eines Haltebolzens 41 am Federgehäuse 19 befestigt.

Das zentrale Zahnrad 30 ist damit, wenn die Sperrklinke 39 in Eingriff ist, über diese Sperrklinke 39 fest mit dem Federgehäuse 19 und damit mit dem kürzeren Schienenarm 9 in einer Richtung gekoppelt. Die Federkraft der Spiralfeder 39 wirkt damit einerseits über den viereckigen Fortsatz 32 auf das zentrale Zahnrad 30 und von diesem über die Sperrklinke 39 und das Federgehäuse 19 auf den kürzeren Schienenarm 9, und andererseits über den Kopplungsstift 39 auf den längeren Schienenarm 10.

Im dargestellten Ausführungsbeispiel versucht die Spiralfeder 29, den kürzeren Schienenarm 9 nach oben, d.h. im Gegenuhrzeigersinn, und den längeren Schienenarm 10 nach unten, d.h. im Uhrzeigersinn, zu verschwenken, so dass auf die Tibia im knienahen Bereich eine Translationskraft in ventraler Richtung ausgeübt wird. Die Vorrichtung stellt damit eine sogenannte PCL-Schiene dar. Es ist erkennbar, dass alternativ hierzu die Aufbringung einer Translationskraft in dorsaler Richtung auf einfache Weise dadurch möglich ist, dass die Spiralfeder 29 und die Sperrklinke 39 in ihrer Wirkungsrichtung umgedreht werden, wodurch die Vorrichtung als ACL-Schiene eingesetzt werden kann.

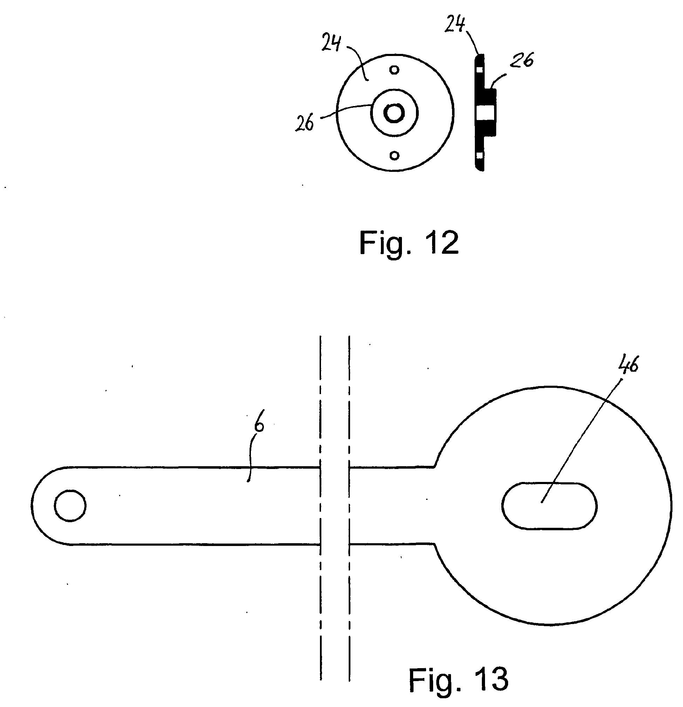

Aus den Figuren 10 und 11 ist eine zweite Ausführungsform der erfindungsgemäßen Vorrichtung ersichtlich, bei der die Oberschenkelschiene 6 im Gelenkbereich ein Langloch 46 aufweist, das sich in Längsrichtung der Oberschenkelschiene 6 erstreckt. Die Oberschenkelschiene 6 ist in Figur 13 in Einzeldarstellung gezeigt. Das Langloch 46 ermöglicht es, dass sich die als Schwenkachse dienende Schraube 23 und damit die gesamte Unterschenkelschieneneinrichtung zusammen mit dem Federgehäuse 19 über eine bestimmte Strecke längs des Langlochs 46 bewegen kann.

Die bei dieser Ausführungsform verwendete Mutter 24 ist in Figur 12 dargestellt. Diese Mutter 24 weist wie die Mutter der ersten Ausführungsform wiederum einen zylindrischen Bund 26 auf, der in das Langloch 46 eingreift und durch die langen Seitenwände des Langlochs 46 mit geringem Spiel geführt wird. Die übrigen Teile der zweiten Ausführungsform sind gleich wie diejenigen der ersten Ausführungsform ausgebildet und werden daher nicht nochmals einzeln beschrieben.

Die Funktionsweise der zweiten Ausführungsform ist identisch zu derjenigen der ersten Ausführungsform. Durch die Kraft der Spiralfeder 29 wird der kürzere Schienenarm 9 in Pfeilrichtung (siehe Figuren 2a bis 2c) zum längeren Schienenarm 10 hin bewegt. Hierdurch wird die Halbschale 13, d.h. die. Unterschenkelschale, um den distalen Kopplungspunkt 17 herum geschwenkt und im kniegelenksnahen Endbereich ventral gedrückt, so dass ein Vorschub der Tibia relativ zum Femur ausgeübt wird.

Dies bewirkt eine Reaktionskraft, welche den Endbereich der Unterschenkelschieneneinrichtung im Gelenksbereich in dorsaler Richtung drückt. Da die Oberschenkelschiene 6 jedoch über die Manschette 7 fest am Oberschenkel fixiert ist, wird der Gelenkbereich der Vorrichtung in fester Relativlage zum Kniegelenk gehalten. Bei Flexion des Kniegelenks versucht jedoch die im Gelenkbereich wirkende Kraft, die Manschette 7 des Oberschenkels geringfügig proximal zu verschieben. Bei anschließender Extension würde dagegen auf die Oberschenkelschiene 6 eine möglicherweise als unangenehm empfundene Zugspannung ausgeübt werden, wenn die Unterschenkelschieneneinrichtung unverschiebbar, wie dies bei der ersten Ausführungsform der Fall ist, an der Oberschenkelschiene gelagert wäre. Das in der Oberschenkelschiene 6 vorgesehene Langloch 46, das sich seitlich zentral neben dem Kniegelenk befindet, lässt jedoch eine Verschiebung der Lagerung längs des Langlochs 46 zu, so dass Zug- oder Druckkräfte in Längsrichtung des Oberschenkels vermieden werden können.

## Patentansprüche

1. Vorrichtung zum Aufbringen einer ventral oder dorsal gerichteten Translationskraft auf einen Unterschenkel (3) im Bereich eines Kniegelenks zur Behandlung oder Nachbehandlung einer Knieinstabilität, insbesondere Kreuzbandinstabilität, mit einer an einem Oberschenkel (1) befestigbaren Oberschenkelschiene (6), einer auf den Unterschenkel (3) einwirkenden Unterschenkelschieneneinrichtung, die gelenkig mit der Oberschenkelschiene (6) gekoppelt und mit einer am Unterschenkel (3) befestigbaren Fixiereinrichtung (12) in Wirkverbindung ist, und mit einer eine bestimmte Vorspannkraft erzeugenden Federeinrichtung, welche auf die Unterschenkelschieneneinrichtung einwirkt, **gekennzeichnet durch** die folgenden Merkmale:
- die Unterschenkelschieneneinrichtung weist einen kürzeren (9) und einen längeren Schienenarm (10) auf, wobei beide Schienenarme (9, 10) relativ zur Oberschenkelschiene (6) schwenkbar sind,
- die beiden Schienenarme (9, 10) sind relativ zueinander schwenkbar angeordnet,
- der kürzere Schienenarm (9) ist an seinem distalen Ende mit der Fixiereinrichtung (12) in einem knienahen Bereich in Kopplungseingriff, während der längere Schienenarm (10) mit seinem distalen Ende mit der Fixiereinrichtung (12) in einem knieferneren Bereich in Kopplungseingriff ist,
- die Vorspannkraft der Federeinrichtung wirkt zwischen dem kürzeren und längeren Schienenarm (9, 10) derart, dass die Schienenarme (9, 10) dazu gedrängt werden, relativ zueinander eine Schwenkbewegung auszuführen, so dass auf die Fixiereinrichtung (12) im knienahen Bereich eine ventral oder dorsal gerichtete Translationskraft aufgebracht wird.

2. Vorrichtung nach Anspruch 1, **dadurch gekennzeichnet, dass** die beiden Schienenarme (9, 10) der Unterschenkelschieneneinrichtung um dieselbe, sich am distalen Ende der Oberschenkelschiene (6) befindende Schwenkachse (11) schwenkbar sind.

3. Vorrichtung nach Anspruch 1 oder 2, **dadurch gekennzeichnet, dass** die Schienenarme (9, 10) an ihren distalen Enden mittels Bolzen (15, 17) in Kopplungseingriff sind, die sich von der Fixiereinrichtung (12) seitlich nach außen erstrecken.

4. Vorrichtung nach einem der vorhergehenden Ansprüche, **dadurch gekennzeichnet, dass** mindestens einer der Schienenarme (9, 10) an seinem distalen Ende ein Langloch (16) aufweist, in das ein Bolzen (15) der Fixiereinrichtung (12) hineinragt, um den Schienenarm (9) längsverschiebbar mit der Fixiereinrichtung (12) zu koppeln.

5. Vorrichtung nach einem der vorhergehenden Ansprüche, **dadurch gekennzeichnet, dass** die am Unterschenkel (2) befestigbare Fixiereinrichtung (12) aus einer Halbschale (13) besteht und die beiden Schienenarme (9, 10) mit der Halbschale (13) in den beiden gegenüberliegenden Endbereichen der Halbschale (13) gekoppelt sind.

6. Vorrichtung nach einem der vorhergehenden Ansprüchen, **dadurch gekennzeichnet, dass** die Federeinrichtung eine Spiralfeder (29) umfasst, die in einem Federgehäuse (19) angeordnet ist, das an einem der Schienenarme (9) befestigt und zusammen mit diesem relativ zur Oberschenkelschiene (6) verschwenkbar ist, wobei die Mittelachse des Federgehäuses (19) mit der Schwenkachse (11) zusammenfällt.

7. Vorrichtung nach Anspruch 6, **dadurch gekennzeichnet, dass** die Vorspannkraft der Federeinrichtung mittels eines Zahnradgetriebes einstellbar ist, das sich im Federgehäuse (19) befindet.

8. Vorrichtung nach einem der vorhergehenden Ansprüchen, **dadurch gekennzeichnet, dass** die Unterschenkelschieneneinrichtung in einem Langloch (46) der Oberschenkelschiene (6) verschiebbar an der Oberschenkelschiene (6) gelagert ist.

9. Vorrichtung nach Anspruch 8, **dadurch gekennzeichnet, dass** sich das Langloch (46) in Längsrichtung der Oberschenkelschiene (6) erstreckt.

## Claims

1. A device for applying a ventrally or dorsally directed translatory force onto a lower leg (3) in the area of a knee joint for treatment or follow-up treatment of knee instability, in particular cruciate ligament instability, with a thigh bar (6) which can be secured on a thigh (1), with a lower-leg bar device which acts on the lower leg (3), is coupled in an articulated manner to the thigh bar (6) and is operatively connected to a fixation device (12) that can be secured on the lower leg (3), and with a spring device which generates a defined pretensioning force and acts on the lower-leg bar device, **characterized by** the following features:
- the lower-leg bar device has a shorter bar arm (9) and a longer bar arm (10), both bar arms (9, 10) being able to swivel relative to the thigh bar (6),
- the two bar arms (9, 10) are arranged so as to be able to swivel relative to one another,
- the shorter bar arm (9) is coupled at its distal end to the fixation device (12) in an area close to the knee, whereas the longer bar arm (10) is coupled with its distal end to the fixation device (12) in an area farther away from the knee,
- the pretensioning force of the spring device acts between the shorter and longer bar arms (9, 10) in such a way that the bar arms (9, 10) are urged to execute a swiveling movement relative to each other, such that a ventrally or dorsally directed translatory force is applied to the fixation device (12) in the area close to the knee.

2. The device as claimed in claim 1, **characterized in that** the two bar arms (9, 10) of the lower-leg bar device are able to swivel about the same swivel axis (11) situated at the distal end of the thigh bar (6).

3. The device as claimed in claim 1 or 2, **characterized in that** the bar arms (9, 10) are coupled at their distal ends by means of bolts (15, 17) which extend laterally outward from the fixation device (12).

4. The device as claimed in one of the preceding claims, **characterized in that** at least one of the bar arms (9, 10) has, at its distal end, an oblong hole (16) into which a bolt (15) of the fixation device (12) protrudes, in order to couple the bar arm (9) to the fixation device (12) in a longitudinally displaceable manner.

5. The device as claimed in one of the preceding claims, **characterized in that** the fixation device (12) which can be secured on the lower leg (2) is made up of a half-shell (13), and the two bar arms (9, 10) are coupled to the half-shell (13) at the two opposite end areas of the half-shell (13).

6. The device as claimed in one of the preceding claims, **characterized in that** the spring device comprises a flat coil spring (29) arranged in a spring housing (19) which is secured on one of the bar arms (9) and, together with the latter, can be swiveled relative to the thigh bar (6), the center axis of the spring housing (19) coinciding with the swivel axis (11).

7. The device as claimed in claim 6, **characterized in that** the pretensioning force of the spring device can be adjusted by means of a toothed wheel gear located in the spring housing (19).

8. The device as claimed in one of the preceding claims, **characterized in that** the lower-leg bar device is mounted in an oblong hole (46) of the thigh bar (6) so as to be displaceable on the thigh bar (6).

9. The device as claimed in claim 8, **characterized in that** the oblong hole (46) extends in the longitudinal direction of the thigh bar (6).

## Revendications

1. Dispositif pour appliquer une force de translation en direction ventrale ou dorsale sur une jambe (3) dans la région d'une articulation du genou pour le traitement ou le post-traitement d'une instabilité du genou, en particulier une instabilité du ligament croisé, comprenant une tringle de cuisse (6) susceptible d'être fixée à une cuisse (1), un système de tringle de jambe agissant sur la jambe (3), qui est couplé de façon articulée avec la tringle de cuisse (6) et qui coopère avec un dispositif de fixation (12) susceptible d'être fixé sur la jambe (3), et comprenant un dispositif à ressort produisant une force de précontrainte déterminée, qui agit sur le système de tringle de jambe, **caractérisé par** les caractéristiques suivantes :
-- le système de tringle de jambe comprend un bras de tringle court (9) et un bras de tringle long (10), les deux bras de tringle (9, 10) étant capables de pivoter par rapport à la tringle de cuisse (6),
-- les deux bras de tringle (9, 10) sont agencés avec possibilité de pivotement l'un par rapport à l'autre,
-- le bras de tringle court (9) est en engagement couplé à son extrémité distale avec le dispositif de fixation (12) dans une région proche du genou, tandis que le bras de tringle long (10) est en engagement couplé à son extrémité distale avec le dispositif de fixation (12) dans une région éloignée du genou,
-- la force de précontrainte du dispositif à ressort agit entre le bras de tringle court et le bras de tringle long (9, 10) de telle façon que les bras de tringle (9, 10) sont forcés à exécuter un mouvement de pivotement l'un par rapport à l'autre, de sorte qu'une force de translation en direction ventrale ou dorsale est appliquée sur le dispositif de fixation (12) dans la région proche du genou.

2. Dispositif selon la revendication 1, **caractérisé en ce que** les deux bras de tringle (9, 10) du système de tringle de jambe sont capables de pivoter autour du même axe de pivotement (11) situé à l'extrémité distale de la tringle de cuisse (6).

3. Dispositif selon la revendication 1 ou 2, **caractérisé en ce que** les bras de tringle (9, 10) sont en engagement couplé à leurs extrémités distales au moyen de goujons (15, 17) qui s'étendent latéralement vers l'extérieur depuis le dispositif de fixation (12).

4. Dispositif selon l'une des revendications précédentes, **caractérisé en ce que** l'un au moins des bras de tringle (9, 10) comporte à son extrémité distale un trou oblong (16) dans lequel pénètre un goujon (15) du dispositif de fixation (12) afin de coupler le bras de tringle (9) avec possibilité de translation longitudinale avec le dispositif de fixation (12).

5. Dispositif selon l'une des revendications précédentes, **caractérisé en ce que** le dispositif de fixation (12) susceptible d'être fixé à la cuisse (12) est formé par une demi-coque (13), et les deux bras de tringle (9, 10) sont couplés avec la demi-coque (13) dans les deux régions terminales opposées de la demi-coque (13).

6. Dispositif selon l'une des revendications précédentes, **caractérisé en ce que** le dispositif à ressort comprend un ressort spiralé (29) qui est agencé dans un boîtier à ressort (19), lequel est fixé à l'un des bras de tringle (9) et capable de pivoter conjointement avec celui-ci par rapport à la tringle de cuisse (6), l'axe médian du boîtier à ressort (19) coïncidant avec l'axe de pivotement (11).

7. Dispositif selon la revendication 6, **caractérisé en ce que** la force de précontrainte du dispositif à ressort est réglable au moyen d'un mécanisme à engrenages qui se trouve dans le boîtier à ressort (19).

8. Dispositif selon l'une des revendications précédentes, **caractérisé en ce que** le système à tringle de jambe est monté sur la tringle de cuisse (6) avec possibilité de translation dans un trou oblong (46) de la tringle de cuisse (6).

9. Dispositif selon la revendication 8, **caractérisé en ce que** le trou oblong (46) s'étend en direction longitudinale de la tringle de cuisse (6).
